# EUROPEAN PATENT APPLICATION

(11) **EP 2 851 081 A1**
(43) Date of publication of application: **25.03.2015**
(21) Application number: 13787415.2
(22) Date of filing: 13.05.2013
(51) Int. Cl.: A61K 36/88, A61K 31/122, A61K 125/00, A61P 33/06

(54) **METHOD FOR PRODUCING A PLANT EXTRACT AND FRACTION, PHARMACEUTICAL COMPOSITIONS AND USE THEREOF**

(30) Priority: 11.05.2012 BR 102012019423
(71) Applicant: Universidade Federal Do Pará, 66075-900 Belém - PA (BR); Universidade Federal de Minas Gerais-UFMG, CEP-31270-901 Belo Horizonte, MG (BR); Museu Paraense Emílio Goeldi, 66040-170 Belém - Pará (BR); Instituto Evandro Chagas, 67030-000 Ananindeua - Pará (BR); Secretaria Executiva De Saúde Pública Do Estado Do, 66015-200 Belém - Pará (BR)
(72) Inventor: DOLABELA, Maria Fâni, 66075-900 Belém - Pará (BR); MARTINS, Michel T., 66075-900 Belém - Pará (BR); BRANDÃO, Dayse Lúcia doNascimento, 66075-900 Guamá (BR); BORGES, Edinilza da Silva, 66075-900 Belém - Pará (BR); PERCÁRIO, Sandro, 66075-900 Belém - Pará (BR); MARINHO, Andrey Moacir do R., 66075-900 Belém - Pará (BR); SILVA, Thiago L. da, 66075-900 Belém - Pará (BR); VILHENA, Thyago da Costa, 66075-900 Belém - Pará (BR); VASCONCELOS, Flávio de, 66075-900 Belém - Pará (BR); MOTA, Eduardo F., 66075-900 Belém - Pará (BR); SILVA, Adriana Veiga da Conceição, 66075-900 Belém - Pará (BR); OLIVEIRA, Alaíde Braga de, 31270-901 Belo Horizonte - Minas Gerais (BR); BRANDÃO, Geraldo Célio, 31270-901 Belo Horizonte - Minas Gerais (BR); PAULA, Renata Cristina de, 31270-901 Belo Horizonte - Minas Gerais (BR); NASCIMENTO, Maria Fernanda Alves do, 31270-901Belo Horizonte - Minas Gerais (BR); PÓVOA, Marinete Marins, 67030-000 Ananindeua - Pará (BR); COELHO-FERREIRA, Marlia Regina, 66040-170 Belém - Pará (BR); BUSMAN, Dâmaris, 66015-200 Belém - Pará (BR)
(74) Representative: Kador & Partner
(86) International application number: PCT/BR2013/000168
(87) International publication number: WO 2013/166576

(57) **Abstract**

The present invention describes the obtaining process of the dichloromethane fraction and subfractions from *Eleutherine plicata,* popularly known as "marupazinho, maruparí, palmeirinha, coquinho, marupaí, marupaú, marupá-piranga, and lírio-folha-de-palmeira". This invention comprises the obtaining process of pharmaceutical compositions that contain the dichloromethane fraction and/or naphthoquinone, as well as its use for malaria treatment. Extract and fractions were assayed and presented antiplasmodial activity, particularly against chloroquine-resistant *Plasmodium falciparum* (clone W2).

## Description

### FIELD OF INVENTION

The present invention describes a process for obtaining an extract and a plant fraction. More particularly, a dichloromethane fraction from the ethanol extract of *Eleutherine plicata,* rich in naphthoquinones. This invention comprises the obtaining process of this fraction as well as the identification of a naphthoquinone. The later was assayed and presented antiplasmodial activity, particularly against chloroquine-resistant *Plasmodium falciparum* (clone W2).

### STATE OF THE ART

*E. plicata* is a tropical America, including the dry fields of brazilian Amazon, native plant popularly known as marupazinho, maruparí, palmeirinha, coquinho, marupaí, marupaú, marupá-piranga, and lírio-folha-de-palmeira. Etnobotanical survey was performed at Igarapé-Miri County (PA, Brasil) through semistructurated interviews and confirmed the usage of marupazinho (*E*. *plicata*). People from this location uses tea made of its bulbs for treatment of diarrhea, amoebiasis, intestinal infection, hepatic diseases, hemorrhages and anemmia (PINTO, L.N. Plantas medicinais utilizadas em comunidades do municipio de Igarapé- Miri /PA: etnofarmácia do Municipio de Igarapé-Miri/PA. 2008. 98 f. Master thesis - Programa de Pós-graduação em Ciências Farmacêuticas, Universidade Federal do Para, Para 2008).

From *Eleutherine* species naphthoquinones were isolated, wherein substances from this class have presented antimalarial activity. An example of *Plasmodium-active* naphthoquinone active is atovacone, synthetic naphthoquinone structurally related to lapachol, a prenylnaphthoquinone common in *Tabebuia* species, genus comprising ipês, South America occurring trees (MIRAGLIA, M.C.M. Estudo quimico de Tabebuia serratifolia (Vahl.) Nichols (Bignoniaceae) e síntese de piranonaftoquinonas, furanonaftoquinonas e antraquinonas. 1991. 326p. Tese de Doutorado. Departamento de Quimica, Universidade Federal de Minas Gerais, Minas Gerais., 1991). Atovacone inhibits the enzyme diidroorotate desidrogenase, whose catalyses the synthesis of uridin monophosphate in parasites.

It is noteworthy that many naphthoquinones, for example the ones isolated from *E. plicata,* have not yet been evaluated in *P. falciparum.* From *E. plicata,* eleuterol and isoeleutherine were already isolated (MALHEIROS, L.C.S. Isoeleutherol and Isoeleutherine: oeleuterol e Isoeleuterina: Potenciais marcadores químicos da tintura de *Eleutherine plicata* Herb. (Iridaceae) e atividades microbiologica e antioxidante Master thesis - Programa de Pós-graduação em Ciências Farmacêuticas, Universidade Federal do Para, Para 2008). Nevertheless, the antiplasmodial activity of the ethanol extract and its fractions were evaluated for the first time by the master thesis of Edinilza Borges, and it is very promising.

Ethanol extract obtained from the bulbs of *E. plicata* and its fractions underwent antiplasmodial activity testing by the technique of HRP-II (NOEDL et al., 2002). Ethanol extract and dichloromethane and ethyl acetate fractions were active, in the assay of diffusion on Agar in the concentration of 500 µg/mL, in S. *aureus.* On the other hand, the methanol fraction was not active in any bacteria (*S. aureus, Pseudomonas aeruginosa* and *Escherichia coli*) and fungus (*Candida albicans*). Active extract and fractions underwent the test of microdilution, being the inhibitory concentration (MIC) of ethanol extract 500 µg/mL, the MIC of ethyl acetate fraction was 250 µg/mL and dichloromethane fraction was 125 µg/mL. Thus, the fractionation improved antimicrobial activity, being the most active fraction and rich in naphthoquinones (dichloromethane fraction). In order to assess the potential bactericide, extract and fractions underwent the test of microdilution followed by sub-culture, where minimum bactericide concentrations (MBC) were higher than 1000µg/mL, suggesting that its antimicrobial activity was of bacteriostatic kind.

Ethanol extract obtained from bulbs of *E. plicata* underwent *A. salina* toxicity assay, counting live and dead animals. After 24h, Probitos analysis method was used to obtain the LD₅₀ value and their confidence intervals (MEYER et al., 1982), with the following result (1000 µg/mL > LD₅₀), indicating that this extract has low toxicity in *A. salina.* The toxicity test in *A. salina* is a widely used biological test as it is fast, reliable, low cost and for showing a good correlation with various biological activities (MEYER et al., 1982)

Patent application JP20080020163 20080131 (A61K31/708; international classification: A61P35/00; C07H1704; A61K36/18) describes the isolation of eleutheroside A of the methanol extract of *Eleutherine palmifolia* (Iridaceae). The compound is useful for the treatment of cancer.

Patent application CN20051119690 20051128 (International classification: A61K36/28; A61K36/748; A61P19/00; A61P29/00; C12G3/02) describes the activities rheumatoid arthritis, rheumatic arthritis, rheumatic dormancy and myalgia, soft tissue contusion and traumatic injury of *Eleutherine plicata.*

Patent application JP20050153747 20050526 (International classification: A23L1/30; A61K36/00; A61K36/18; A61 K36/81; A61P25/00; A61P3/02) provides a new fatigue recovery agent, this is characterized by the presence of at least one species of plant in the genus *Eleutherine* (Iridaceae), a plant of the genus *Solanum* (Solanaceae), a plant of the genus *Eucalyptus* (Myrtaceae) and a plant of the genus *Tabebuia* (Bignoniaceae) and/or an extract of the plant as an active ingredient.

Patent application JP20040071888 20040315/application WO2005JP04415 20050314 (international classification: A23L1/30; A61K36/00; A61K36/185; A61 K36/47; A61 K36/48; A61 K36/53; A61 K36/77; A61 K36/85; A61K36/88; A61P13/08; A61P43/00; C12N9/99; (IPC1-7): A23L1/30; A61 K35/78; A61P13/08; A61P43/00; C12N9/99 e European: A23L1/30B; A61K36/185; A61 K36/47; A61 K36/48; A61 K36/53; A61 K36/77; A61 K36/85; A61 K36/88) intended to provide a new testosterone 5-alpha-reductase inhibitor and a composition for inhibiting prostatic hypertrophy. This product must contain one or more plants belonging to the genera, *Allophylus, Archidendron, Butea, Dianella, Eleutherine, Horsfieldia, Meliosma, Orthosiphon, Phyllanthus, Salacia* and *Vitex.*

The patent application JP20020266273 20020912 (international classification: A23L1/30; A23L2/38; A23L2/52; A61K36/00; A61K36/02; A61K36/18; A61 K36/81; A61P3/04; (IPC1-7): A23L1/30; A23L2/38; A23L2/52; A61 K35/78; A61K35/80; A61P3/04) aimed to provide a food and a drink for vegetable fat reduction, being effective in the prevention and treatment of obesity and related diseases. This product consists of algae, plants or extracts *(Tabebuia, Eleutherine, Solanum,* Myrtaceae and *Eucalyptus*).

In addition to these documents, it is worth mentioning that *E. plicata* is on the national list of medicinal plants of interest to the public health system (Brazil), but there is no allegation of popular use for malaria and feverish diseases.

### PROBLEMS FROM THE ART

*Plasmodium falciparum* has quickly acquired resistance to the drugs available. Chloroquine was widely used for the treatment of *falciparum* malaria and the justifications for the use of chloroquine were: the drug has low toxicity and is economically viable.

Due to the growing resistance of the parasites to chloroquine, new drugs had to be introduced, such as artemisinin, artemisinin derivatives and atovacone. However, *P. falciparum* resistance to artemisinin derivatives has been recently described. Therefore, there is a need for new antimalarial agents that are effective against chloroquine-resistant *P. falciparum* strains, promoting healing within a reasonable period of time to ensure adherence to treatment, besides being safe and cost-effective (FIDOCK, D.A.; ROSENTHAL, P.J.; CROFT, S.L.; BRUN, S. e NWAKA, S. Antimalarial drug discovery: efficacy models for compound screening. Nat Rev Drug Discov, Jun 2004; 3(6): 509-20).

### ADVANTAGES OF THE TECHNOLOGY

The plant from which the dichloromethane fraction is obtained is easy to cultivate. The extractive process for obtaining the ethanol extract is simple, as well as obtaining the fraction and active subfractions. Thus, it would be easy to carry on studies, to obtain a standardized fraction and to develop a phytotherapic.

### BRIEF DESCRIPTION OF THE FIGURES OF FIGURES

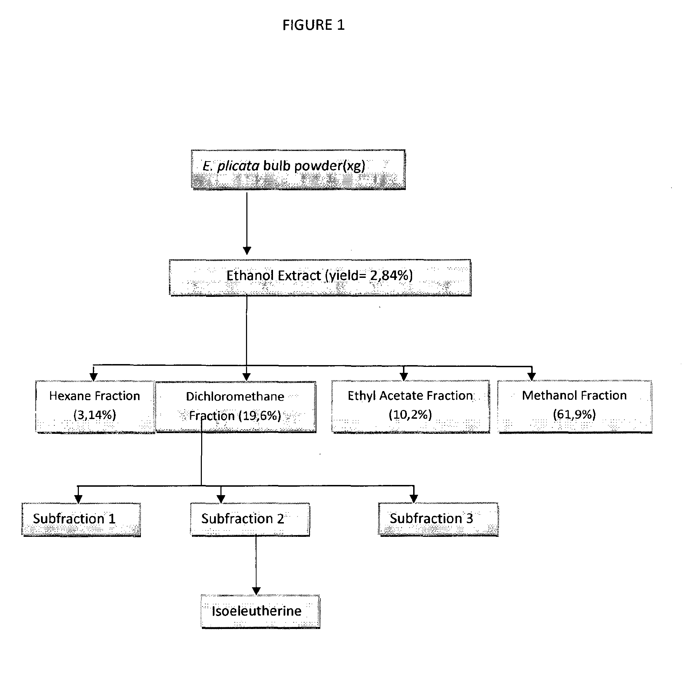

Figure 1 shows process for obtaining the dichloromethane fraction and subfractions thereof.

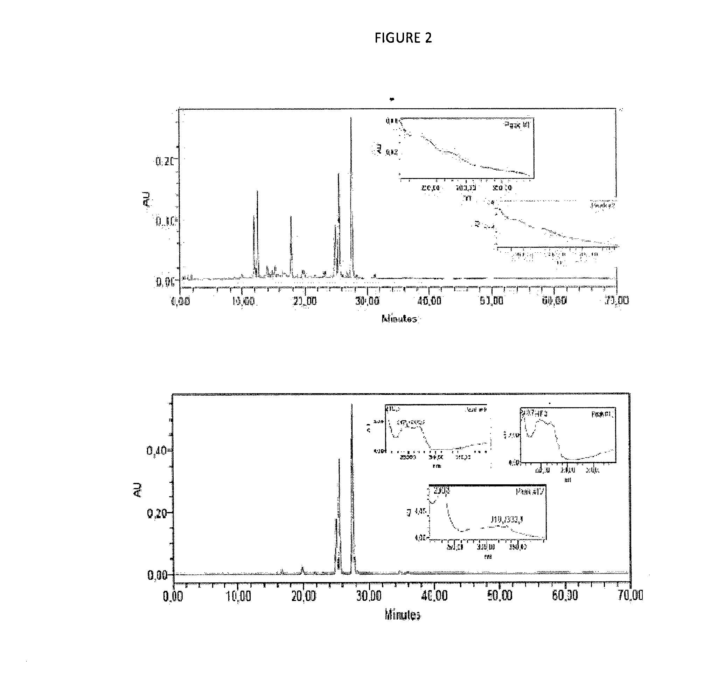

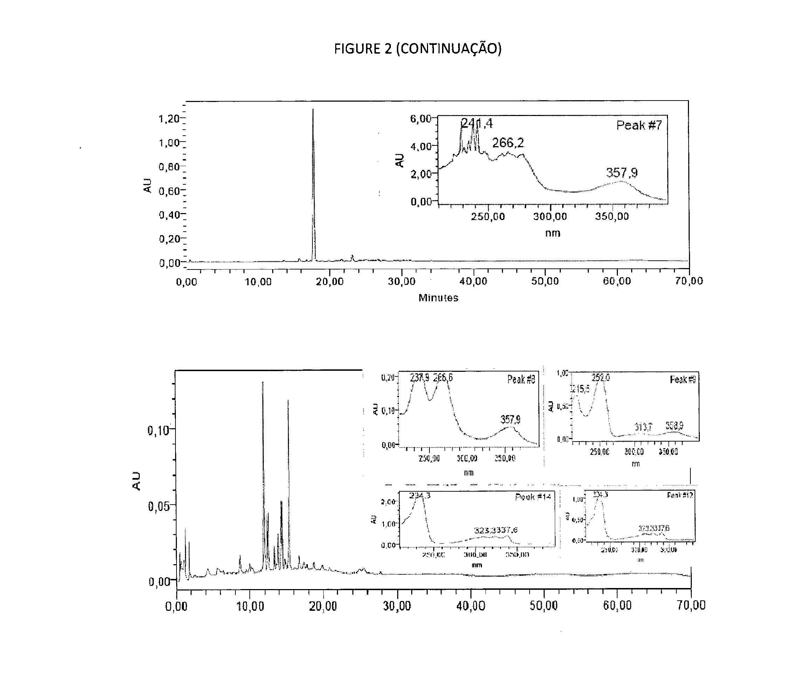

Figure 2 shows chromatograms of the ethanol extract obtained from bulbs of *E*. *plicata* and fractions thereof. Condition at A: RP-18 column, Mobile phase: Time 0min- 95% water, 5% acetonitrile; Time 65min- 5% water, 95% acetonitrile; Time 70min- 5% water, 95% acetonitrile; Temperature= 40° C, Flow= 1 mL/min; Detection= 350nm.

Grinding for 7 days with alcohol at 96°.

Filtering and concentration at reduced pressure.

10g extract: Stationary phase column chromatography: silica gel

Mobile phases: increasing polarity

20mg preparative layer chromatography

Eluent: dichloromethane

Label: a- ethanol extract from *E. plicata* bulbs; b- dichloromethane fraction; c- ethyl acetate fraction; d- methanol fraction.

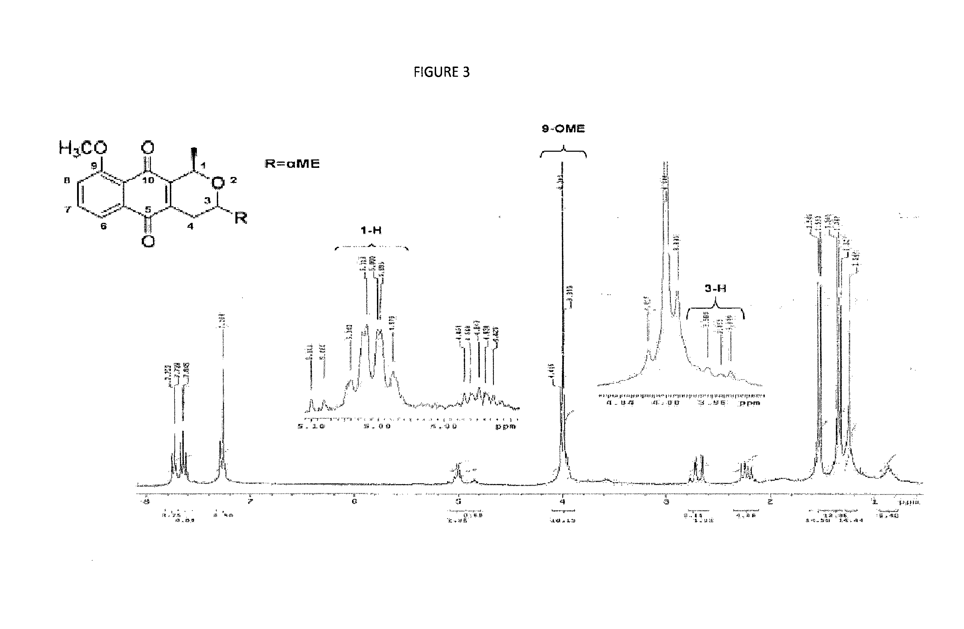

Figure 3: shows the nuclear magnetic resonance analyzes of subfraction 2.

### DETAILED DESCRIPTION OF TECHNOLOGY

This invention describes a process for obtaining an extract and a naphthoquinones-rich dichloromethane fraction from *Eleutherine plicata* plant species in addition to isolating and purifying isoeleutherine, naphthoquinone present on said plant species. The invention further comprises the evidence of antiplasmodial activity of this extract/fraction. The invention shows that the purification process has contributed to the increased antiplasmodial activity, and consequently the fraction obtained presents increased activity regarding the extract for malaria treatment.

The present invention can be better understood through the following examples, which are not limiting of the technology.

### 1. Obtaining of ethanol extract from bulbs of E. plicata, fractions and subfractions

Sprayed bulbs of *E. plicata* underwent extraction thru maceration (7 days), with ethanol at 96°GL. Subsequently, the dye was concentrated in the rotating evaporator at 50°C, under reduced pressure, until a residue was formed, being held in a desiccator until a constant weight was reached.

Ethanol extract from bulbs of *E. plicata* (EEBEP) (5.0g) underwent fractionation on a chromatographic column (size = 69.3cm and diameter = 4cm). As stationary phase, silica gel was used (flesh the 0.063 to 0.2mm) and, as mobile phase, the solvents: hexane (1 L), dichloromethane (1 L), ethyl acetate (1 L) and methanol (1 L). Fractions were concentrated in the rotating evaporator (50°C, under reduced pressure, until a residue was formed, being held in a desiccator until a constant weight was reached.

The extract and fractions were subject to studies on High Performance Liquid Chromatography (HPLC-DAD), and the UV spectra obtained demonstrate the presence of quinines. The extract has a high chemical complexity, but the dichloromethane fraction shows 3 major peaks. Similar fact was observed through thin-layer chromatography, and ultraviolet spectrometry analysis suggest the presence of quinones.

Dichloromethane fraction underwent fractionation by chromatography on preparative layer, 3 subfractions being obtained, named S1, S2, and S3. The S1, S2, and S3 fractions were subjected to studies on NMR, being identified the S2 fraction as high-purity isoeleutherine demonstrating the efficiency of the adopted process. Identification of S1 and S3 were not yet completed.

S2 fraction actually consists mostly of isoleutherine, a naphthoquinone already isolated from *E. plicata* (MALHEIROS, Isoeleutherol and Isoeleutherine : L.C.S. Potential chemical markers of Eleutherine plicata Herb dye (Iridaceae) and microbiological and antioxidant activities. 2008.67 p. Master thesis - Pharmaceutical Sciences - Pharmacy College, Universidade Federal do Para, Para 2008). On the NMR1 H obtained from this fraction, it is noted that hydrogens 6H, 7H, and 8H of the aromatic ring appear on δ 7.739, δ 7.677, and δ 7.26. The hydrogens 1-Me and 1-H appears as duplets on δ 1.33 and δ1.54, respectively and the hydrogen 3-Me appear as duplets on δ 1.34.

### 2. Evaluation of antiplasmodial activity of the extract and its fractions.

Regarding the antiplasmodial activity of the ethanol extract of *E. plicata,* by means of the characteristic presented CLAE profile, a high activity was observed, however, fractionation led to isolation of a more active fraction, the dichloromethane fraction and two fractions with less antiplasmodial activity (ethyl acetate and methanol; Table 1).

**Table 1: Antiplasmodial activity of ethanol extract obtained from E. plicata bulbs and fractions thereof**

| Sample | Mean Inhibitory concentration (µg/mL) |
|---|---|
| Ethanol Extract | 6.57 |
| Dichloromethane Fraction | 2.87 |
| Ethyl Acetate Fraction | 16.38 |
| Methanol Fraction | >50 |

These results suggest that naphthoquinones are the pharmacological markers. This assessment used the methodology described by NOEDL, H; WERNSDORFER, W. H; MILLER, R.S; WONGSRICHANALAI, S. Histidine-Rich Protein II: a Novel Approach to Malaria Drug Sensitivity Testing. Antimicrobial Agents and Chemotherapy, v. 46, n. 6, p. 1658-1664, 2002. The ethanol extract and dichloromethane fraction were active regarding *P. falciparum* (Cl₅₀ < 10µg/mL), the later being more active; the ethyl acetate fraction was moderately active (10µg/mL < Cl₅₀ < 50µg/mL) and the methanol fraction was considered inactive (Cl₅₀> 50µg/mL).

A second object of the invention relates to pharmaceutical compositions comprising the ethanol extract of *Eleutherine plicata* or its dichloromethane fraction. Additionally, the referred composition can further comprise or not an isoleutherine obtained e derivatives thereof. More particularly, said compositions comprise a standardized fraction rich in naphthoquinones and at least one pharmaceutical and pharmacologically acceptable excipients.

The referred composition have antiplasmodial activity and can be used on the preparation of a medicament for malaria treatment. Said medicament is preferably administered to the individual in need of a treatment by oral, intramuscular, intravenous, intraperitoneal, subcutaneous, transdermic routes. Alternatively, referred medicament can be administered by means of devices that can be implemented or injected in the patient. Particularly, the preferred administration route is oral.

## Claims

1. Process for obtaining a plant dichloromethane extract and fraction rich in naphthoquinones from *Eleutherine plicata* plant species **characterized by** comprising:
a. Grinding extraction of the sprayed *E. plicata* bulbs(7 days) with ethanol at 96 °GL;
b. Concentration in rotatory evaporator under reduced pressure until a residue is formed, being held until a constant weight is obtained;
c. Fractioning of the *E. plicata* bulb ethanol extract (EEBEP) in chromatography column having silica gel as stationary phase and, as mobile phase, the solvents: hexane, dichloromethane, ethyl acetate e methanol;
d. Concentration of the fraction on a rotatory evaporator under reduced pressure until a residue is formed, being held until a constant weight is obtained.

2. Process according to claim 1 **characterized by** the extract and fractions obtained being additionally purified and presenting an increase on antiplasmodial activity.

3. Process according to claim 1 **characterized by** the extract and fractions obtained having quinones.

4. Process according to claim 1 **characterized by** the dichloromethane fraction having isoleutherine at high purity levels.

5. Process according to claim 1 **characterized by** the ethanol extract and the dichloromethane fraction being active against *P.falciparum* (Cl₅₀<10µg/mL), the later being more active ; the ethyl acetate fraction moderately active (10µg/mL< Cl₅₀< 50µg/mL) and the methanol fraction considerably inactive (Cl₅₀> 50µg/mL).

6. Pharmaceutical compositions **characterized by** comprising the ethanol extract of *Eleutherine plicata* or its dichloromethane fraction and at least a pharmaceutical and pharmacologically acceptable excipient.

7. Pharmaceutical compositions according to claim 6 **characterized by** further comprising or not an isoleutherine obtained and derivatives thereof.

8. Pharmaceutical compositions according to claim 6 **characterized by** comprising a standardized fraction rich in naphthoquinones and at least a pharmaceutical and pharmacologically acceptable excipient.

9. Pharmaceutical compositions according to claim 6 **characterized by** having antiplasmodial activity.

10. Use of the pharmaceutical compositions according to claims 6 to 9 **characterized by** being in the preparation of a medicament for treatment of malaria.

11. Use according to claim 10 **characterized by** being preferably administered to the individual in need of treatment by oral, intramuscular, intravenous, intraperitoneal, subcutaneous, transdermic routes.

12. Use according to claim 10 **characterized by** the medicament being alternatively administered by means of devices able to be implemented or injected.
